# EUROPEAN PATENT SPECIFICATION

(11) **EP 4 170 045 B1**
(45) Date of publication and mention of the grant of the patent: **24.12.2025**
(21) Application number: 22733275.6
(22) Date of filing: 11.02.2022
(51) Int. Cl.: C12Q 1/6848, C12Q 1/6806

(54) **METHOD FOR SEPARATING NUCLEIC ACID AMPLIFICATION SYSTEM, AND SEPARATOR**
VERFAHREN ZUR TRENNUNG EINES NUKLEINSÄUREAMPLIFIKATIONSSYSTEMS UND SEPARATOR
PROCÉDÉ DE SÉPARATION D'UN SYSTÈME D'AMPLIFICATION D'ACIDE NUCLÉIQUE, ET SÉPARATEUR

(43) Date of publication of application: 26.04.2023
(73) Proprietor: DA AN GENE CO., LTD., Guangdong 510665 (CN)
(72) Inventor: JIANG, Xiwen, Guangzhou, Guangdong 510665 (CN); LIU, Aishan, Guangzhou, Guangdong 510665 (CN); WANG, Zhouquan, Guangzhou, Guangdong 510665 (CN); ZHENG, Sangsang, Guangzhou, Guangdong 510665 (CN)
(74) Representative: Petraz, Gilberto Luigi
(86) International application number: PCT/CN2022/075964
(87) International publication number: WO 2022/135616

(56) References cited:
- EP-A1- 2 682 480
- CN-A- 101 787 347
- CN-A- 106 065 351
- CN-A- 109 957 612
- CN-A- 111 548 918
- CN-A- 111 548 918
- CN-U- 207 567 206
- CN-U- 207 567 206
- KRUPA ET AL: "Phase change materials based on low-density polyethylene/paraffin wax blends", EUROPEAN POLYMER JOURNAL, PERGAMON PRESS LTD OXFORD, GB, vol. 43, no. 11, 29 October 2007 (2007-10-29), pages 4695 - 4705, XP022318824, ISSN: 0014-3057, DOI: 10.1016/J.EURPOLYMJ.2007.08.022

## Description

### TECHNICAL FIELD

The present invention relates to the technical field of molecular biology, and particularly, to a method for separating a nucleic acid amplification system and a separating agent.

### BACKGROUND

Nucleic acid amplification test is a method in which nucleic acid sequences to be detected are first amplified by enzymes prior to the detection. It mainly includes polymerase chain reaction (PCR), PCR direct sequencing, *in-situ* PCR, telomere repeat amplification protocol, *etc.* Nucleic acid amplification technologies are widely used in clinical microbiology, blood screening, genetic disease diagnosis and prevention, forensic medicine and other fields. These technologies are an important part of emerging pharmacogenomics, prenatal diagnostics, and molecular-level diagnostics and treatments.

PCR is a common technology for nucleic acid amplification and is widely used in detection of pathogens, infectious diseases, and genetic diseases. The PCR is usually composed of the following components: a reaction buffer mixture (containing dNTP, magnesium ions, and a buffer solution), enzymes (a reverse transcriptase, a Taq enzyme, a UDG enzyme, an RNA enzyme inhibitor, *etc.*)*,* primers, a probe, and a sample solution. Since the buffer solution contains all the components required for activating the enzymes, mixing the enzymes with buffer solution, primers and the probe during storage could affect the stability of the primers and the probe, which prevents the enzymes from long-term shelf-life. Therefore, the above mentioned components in conventional PCR kits are usually stored separately in different tubes, and only mixed and aliquoted when in use, by adding and mixing with the sample solution followed by immediate reaction. Because of the high sensitivity, the PCR preparation process needs to be performed in a preparation room with Class 100,000. The buffer solution and the enzymes are kept at a low temperature after mixing, and aliquoted into PCR tubes, and then samples are added instantly for reaction. Particularly for high-throughput tests, the preparation and aliquoting processes have a larger workload, the reaction system is prone to contamination, and errors are more likely between different batches and tubes of reaction. Moreover, certain technical skills are required for the operators to aliquot enzymes and add samples, and otherwise it is likely to cause damages and contamination to the enzymes which can be costly.

To simplify this step, the commonly used methods are: (1) the buffer solution and the enzymes are first aliquoted into the PCR tubes in a mixed form, and then lyophilized to produce single-tube reagents. When in use, a resuspended solution containing primers and probes is added with samples, but this solution still requires steps of solution addition, mixing and tube cap opening for twice, and the cost for lyophilization is high. (2) The buffer solution, the primers and the probe are aliquoted into single tubes in a mixed form, and an enzyme solution and samples are then added when in use. This solution also requires the steps of solution addition, mixing and tube cap opening for twice. It is obvious that the conventional commonly used single-tube formulations of PCR reagents still require the steps of solution addition, mixing and tube cap opening for twice, thus, are still not simplified enough to minimize the workload for the high-throughput test. In addition, the requirements for the clean environment to prepare the reaction system, and the improper operation can easily cause contamination, which proves that it does not comply to rapid on-site detection.

Therefore, to simplify the operations, reduce the technical skill requirements and workload for the operators, and reduce PCR contamination is very important for nucleic acid detection process. In particular, it is of great practical significance for large -scale tests and high-throughput tests upon the rapid spread of epidemiological and infectious pathogens. The Chinese invention patent, CN101787347B, provides a reaction tube for reaction process control through temperature. By designing sealing layers with different melting points in the tube, a required reaction reagent is then sealed in the sealing layers. Melting of a sealing material is realized by changing the temperature to release the sealed reaction reagent to realize the control over the reaction process. One-tube reaction reagents are realized, and the steps are simplified. However, this technology also has obvious limitations. The sealing layer realizes the dissolution of the separating layer by changing of the temperature, and thus the applicability thereof has obvious limitations. Different nucleic acid amplification enzymes and system solvents have different sensitivity to temperature. Moreover, the dissolution of the sealing layer takes a certain amount of time, and the uneven dissolution will easily affect the subsequent amplification reaction; meanwhile, the operation of dissolving the sealing layer at a relatively high temperature for a long time will more or less irreversibly affect the components in the nucleic acid amplification system. CN111548918A discloses a barrier for separating reagents of a nucleic acid amplification suitable for a hot-start approach.

Therefore, it is imperative to develop a technology that can realize the one-pot nucleic acid amplification reaction system that can be mixed simply and effectively during use, without affecting the performance of system reagents.

### SUMMARY

In view of the above existing problems, the present invention aims to provide a method for separating a nucleic acid amplification system and a separating agent, and provides a paraffin separating agent suitable for the method. A buffer solution, a primer-probe mixed solution and an enzyme solution in the nucleic acid amplification system can be effectively separated in one same container. When in use, only a sample to be tested is added, and after capping the separating agent is ruptured under a certain external force (such as centrifugation, which is a conventional operation during configuration of the amplification system, and time required is extremely short), so that PCR reaction components can be mixed, and then directly subjected to on-computer test. By using the separating agent and the method of the present invention, only one step of sample addition and tube capping is needed, and operations can be minimized, thus, the process is simplified and the time is saved. Additionally the amplification contamination probability can be reduced; and technical skill requirements for operators are greatly reduced (the entire detection process only requires: cap opening - sample addition - capping - centrifuging - on computer test).

The primary purpose of the present invention is to provide a method for separating a nucleic acid amplification system.

Another purpose of the present invention is to provide a separating agent for a nucleic acid amplification system.

The above-mentioned purposes of the present invention are achieved through the following technical solutions.

The present invention provides a separating agent for a nucleic acid amplification system, including polyethylene wax, solid paraffin and liquid paraffin.

The above separating agent of the present invention is used in combination with a specific type of paraffin. Under a condition of a simulated transportation process (-20°C to room temperature, vortex for 10 seconds at 3,000 rpm), a separating layer is intact without rupture, but it is quickly ruptured under specific external force conditions, and separated reaction reagents are released and the components of PCR system are mixed, which is convenient for direct use in subsequent nucleic acid amplification procedures.

Preferably, a mass ratio of the polyethylene wax to the solid paraffin to the liquid paraffin is: (0.02 - 0.05) : (0.6 - 1.2) : (4 - 10).

More preferably, a mass ratio of the polyethylene wax to the solid paraffin to the liquid paraffin is: (0.03 - 0.05) : (0.6 - 0.8) : (4 - 10). After a PCR amplification system is separated by utilizing a paraffin formula in this ratio in a centrifuge tube, the separating layer can be kept intact without rupture under the condition of the simulated transportation, but it is ruptured under specific centrifugal action. After rupture, it can be completely dissolved at an initial temperature set by a PCR program, and does not affect the fluorescent signals of PCR, which meets the performance requirements for PCR reagent separation.

Preferably, a molecular weight of the polyethylene wax is 1,000 to 10,000.

Further preferably, a molecular weight of the polyethylene wax is 4,000 to 6,000.

Preferably, a number of carbon atoms of the solid paraffin is 9 to 30.

Further preferably, the number of carbon atoms of the solid paraffin is 20 to 25.

Preferably, a number of carbon atoms of the liquid paraffin is 9 to 13.

Further preferably, the number of carbon atoms of the liquid paraffin is 9 to 11.

The present invention further provides a method for separating a nucleic acid amplification system, in which the above separating agent is utilized as a separating layer to separate the components from mixing with each other in one same container; and the separating layer can be ruptured by applying an external force to mix the nucleic acid amplification system.

The present invention further provides a method for controlling mixing of a nucleic acid amplification system by an external force, in which the above mentioned separating agent is utilized as a separating layer to separate reagents in the nucleic acid amplification system in one same container, and the separating layer is ruptured by applying the external force to realize mixing of the nucleic acid amplification system. The applying the external force is to apply an external force matching a destructive force that the separating layer can withstand to crush a paraffin mixture separating layer.

Preferably, the separating layer can be vortexed for 10 to 20 seconds at 3,000 rpm on a vortex oscillator without rupture.

The applying the external force can crush the separating layer by centrifuging, oscillating or other external force that will not damage the container.

Preferably, the external force is a centrifugal force.

Preferably, the applying the external force is to centrifuge for 5 to 60 seconds under action of a centrifugal force no less than 5,000 g.

Further preferably, the applying the external force is to centrifuge for 10 to 30 seconds at 5,000 g to 10,000 g.

Preferably, the nucleic acid amplification system is a PCR amplification reaction system.

As a preferred implementable mode, a separating method of single-tube PCR reagents is as follows:
A PCR 8-tube strip is taken, and added with a PCR buffer solution (containing a primer, a probe and dNTP) to a bottom of the PCR 8-tube strip; the above paraffin separating agent is taken, and heated to be melted and then added into the PCR 8-tube strip to make paraffin float on a surface of the buffer solution to be solidified; finally an enzyme solution is added, and a tube cap is put on, that is, separation of PCR reagents and preparation of single-tube reagents are completed.

Compared to the prior methods, the present invention has the following advantages.

The present invention uses a specially formulated paraffin mixture as a separating agent, so the buffer solution, primer-probe mixed solution and enzyme solution in the PCR system can be effectively separated in the same tube. When in use, only the sample to be tested is added, and after capping, an external force that matches the separating agent's tolerance is applied to rupture the separating agent, to mix the components of PCR system. By using the separating agent as well as the methods in the present invention, only one step of sample addition and tube cap opening are needed, operations are minimized, the chance of amplification reaction system contamination is reduced, and the separating agent of the present invention does not affect the PCR performance.

### BRIEF DESCRIPTION OF DRAWINGS

FIG. 1 is a schematic diagram of separation of PCR reagents in a single-tube .
FIG. 2 shows PCR results, in which curves without asterisks are for N gene amplification; and curves with asterisks are for lab gene amplification.

### DETAILED DESCRIPTION OF EMBODIMENTS

The present invention is further illustrated below in combination with specific embodiments, but examples do not limit the present invention in any form. Unless otherwise specified, raw material reagents used in the examples of the present invention are conventionally purchased raw material reagents.

### Example 1 Different formulas of paraffin separating agents

This example provides a series of different formulas of paraffin separating agents, as shown in Table 1 and Table 2 for details.

**Table 1 Mixed paraffin separating agents with different ratios**

| Formulas of separating agents | Polyethylene wax (g) | Solid paraffin (g) | Liquid paraffin (ml) |
|---|---|---|---|
| 1 | 0.02 | 0.6 | 4 |
| 2 | 0.02 | 0.8 | 6 |
| 3 | 0.02 | 1.0 | 8 |
| 4 | 0.02 | 1.2 | 10 |
| 5 | 0.03 | 0.6 | 6 |
| 6 | 0.03 | 0.8 | 4 |
| 7 | 0.03 | 1.0 | 10 |
| 8 | 0.03 | 1.2 | 8 |
| 9 | 0.04 | 0.6 | 8 |
| 10 | 0.04 | 0.8 | 10 |
| 11 | 0.04 | 1.0 | 4 |
| 12 | 0.04 | 1.2 | 6 |
| 13 | 0.05 | 0.6 | 10 |
| 14 | 0.05 | 0.8 | 8 |
| 15 | 0.05 | 1.0 | 6 |
| 16 | 0.05 | 1.2 | 4 |
| 17 | 0.01 | 0.8 | 4 |
| 18 | 0.06 | 0.6 | 10 |
| 19 | 0.03 | 0.5 | 4 |
| 20 | 0.05 | 1.4 | 10 |
| 21 | 0.03 | 0.8 | 2 |
| 22 | 0.05 | 0.6 | 12 |

In Table 1, the polyethylene wax is polyethylene wax with a molecular weight of 4,000 to 6,000; the solid paraffin is solid paraffin with the carbon atom number of 20 to 25; and the liquid paraffin is liquid paraffin with the carbon atom number of 9 to 11.

**Table 2 Mixed paraffin separating agents with different raw materials**

| Formulas of separating agents | Polyethylene wax (0.05 g) | Solid paraffin (0.6 g) | Liquid paraffin (10 mL) |
|---|---|---|---|
| 23 | The molecular weight is 1,000 to 4,000 | The number of carbon atoms is 20 to 25 | The number of carbon atoms is 9 to 11 |
| 24 | The molecular weight is 6,000 to 10,000 | The number of carbon atoms is 20 to 25 | The number of carbon atoms is 9 to 11 |
| 25 | The molecular weight is 4,000 to 6,000 | The number of carbon atoms is 9 to 20 | The number of carbon atoms is 9 to 11 |
| 26 | The molecular weight is 4,000 to 6,000 | The number of carbon atoms is 25 to 30 | The number of carbon atoms is 9 to 11 |
| 27 | The molecular weight is 4,000 to 6,000 | The number of carbon atoms is 20 to 25 | The number of carbon atoms is 11 to 13 |

### Example 2 Performance test of separating PCR reagents by different formulas of paraffin separating agents

### 1. Separating method

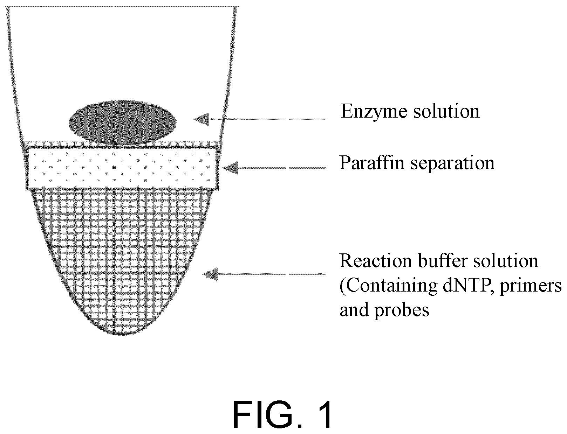

A schematic diagram of separation of single-tube PCR reagents is shown in FIG. 1. Specific steps are as follows:
(1) a PCR 8-tube strip was used, and 15 uL of a PCR buffer solution (containing primers, probes and dNTP) was added to a bottom of the PCR 8-tube strip;
(2) 27 formulas of paraffin separating agents in Example 1 were used and heated to 100°C for melting, then 10 uL of paraffin separating agents was added to each tube, and paraffin floated on a surface of the buffer solution and was solidified; and
(3) a small amount of bromophenol blue was added into an enzyme solution until blue color appeared, 5 uL of the enzyme solution was added to the solidified paraffin layer, the tube was capped, and preparation of the single-tube reagents was completed.

### 2. Stability test

(1) The above prepared 27 formulas of single-tube reagents were vortexed for 10 seconds at 3,000 rpm to simulate a transportation process, and the leakage situation was inspected;
(2) The above prepared 27 formulas of single-tube reagents were put into a centrifuge and centrifuged for 20 seconds with a centrifugal force of 5,000 g, and the leakage situation was inspected; and
(3) The above prepared 27 formulas of single-tube reagents were placed into a thermostatic metal bath and heated for 2 minutes at 55°C, and the melting situation was inspected, wherein complete melting is considered when a paraffin layer is completely transparent.

### 2. Test results

Leakage and melting results are shown in Table 3.

**Table 3**

| Test indexes of different formulas of separating agents | Oscillation leakage | Centrifuging leakage | Heating and melting at 55°C |
|---|---|---|---|
| 1 | - | + | - |
| 2 | - | + | - |
| 3 | - | + | - |
| 4 | - | + | - |
| 5 | - | + | + |
| 6 | - | + | + |
| 7 | - | + | - |
| 8 | - | + | - |
| 9 | - | + | + |
| 10 | - | + | + |
| 11 | - | + | - |
| 12 | - | + | - |
| 13 | - | + | + |
| 14 | - | + | + |
| 15 | - | + | - |
| 16 | - | + | - |
| 17 | + | + | + |
| 18 | + | + | + |
| 19 | + | + | + |
| 20 | + | + | + |
| 21 | + | + | - |
| 22 | + | + | - |
| 23 | - | - | + |
| 24 | - | + | - |
| 25 | - | - | + |
| 26 | - | + | - |
| 27 | - | - | + |
| "+" means leakage or melting, and "-" means no leakage or melting. | | | |

The test results in Table 3 demonstrates that separating agents 1 to 16 did not result leak after severe vortexing, indicating that they can endure oscillation during transportation without rupture; moreover, after centrifuging for 20 seconds at 5,000 g, a separating layer could be ruptured to mix upper and lower layers of solutions, indicating that even mixing can be facilitated during use; in addition, separating layers with agents 5, 6, 9, 10, 13 and 14 could be completely melted after heating for 2 minutes at 55°C, and could be directly used for nucleic acid amplification procedures at an initial temperature of 55°C and below. Separating layers with agents 1, 2, 3, 4, 7, 8, 11, 12, 15 and 16 cannot be completely melted after heating for 2 minutes at 55°C, but they can be rapidly and completely melted when the temperature reaches 60°C. They can also be suitable for nucleic acid amplification procedures at other initial temperatures and reagent separation of the nucleic acid amplification system. In summary, the melting temperatures of different separating layers are slightly different, and therefore, applicable nucleic acid amplification procedures are also different.

In addition, although separating layers with agents 17 to 22 could be ruptured after centrifuged for 20 seconds at 5,000 g to mix the upper and lower layers of solutions, leaking situation was observed after vortexing experiment, and therefore, these agents are not suitable for long-distance transportation.

Separating layers with agents 23, 25 and 27 did not leak after severe vortexing, and were not ruptured completely after centrifuged for 20 seconds at 5,000 g, but they could be ruptured completely after centrifuged for 20 seconds under a centrifugal force increased to 6,000 g; separating agents 24 and 26 did not leak after severe vortexing, and could not be melted completely after heated for 2 minutes at 55°C, but they could be rapidly and completely melted after a temperature rose to 60°C, thus, can also satisfy the reagent separation requirements of the nucleic acid amplification system.

### Application Example Preparation of PCR reagents for rapid novel coronavirus detection by using paraffin separating agents

In this Application Example, separating reagents 1, 3, 6, and 13 in Example 1 were used as examples to separate the PCR reagents for detection of novel coronavirus (2019-nCoV).

### 1. Experimental method

A novel coronavirus (2019-nCoV) nucleic acid detection kit (a PCR-fluorescent probe method) is manufactured by DAAN GENE, SUN YAT-SEN UNIVERSITY. According to the separating method in Example 2, separating agents 1, 3, 6 and 13 were used to prepare single-tube reagents. The reagents without separating agents were used as a control group, a positive reference template of 106-103copies/ml was detected, and PCR procedures were as follows:
2 minutes at 55°C; 2 minutes at 95°C; (30 seconds at 95°C, 30 seconds at 65°C to read fluorescence) × 40 cycles.

### 2. Experimental results

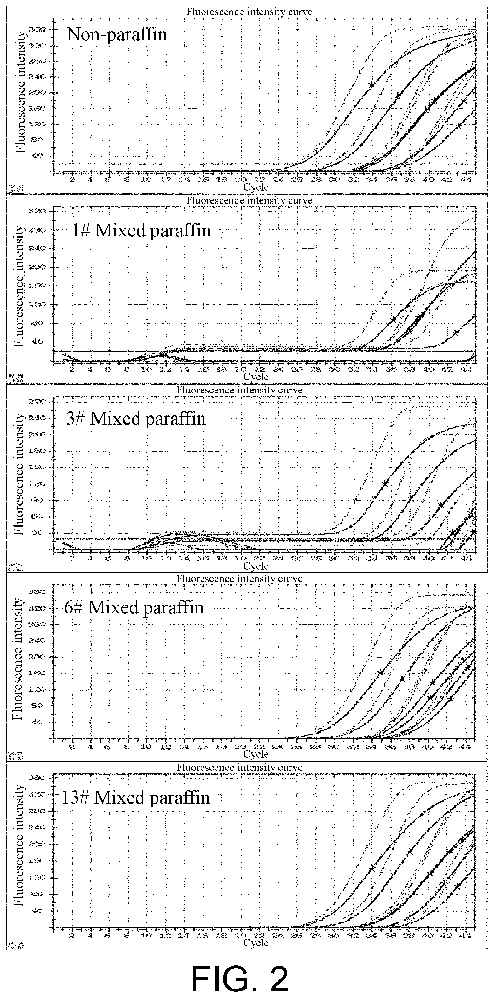

FIG. 2 displays the PCR results showing that amplification curves using mixed paraffin separating agent groups 1 and 3 demonstrated significant fluctuations with respect to a baseline during the first 20 cycles, resulting in wrong interpretation of ct values. This can be explained that paraffin was not completely melted at 55°C, affecting penetration of fluorescence. Fluorescence values and ct values of amplification curves of mixed paraffin separating agent groups 6 and 13 were basically the same as those of a non-paraffin group, indicating that separating agents did not affect the performance of PCR and met performance requirements of separation of PCR reagents.

The inventor further prepared the mixed paraffin separating agent group 1 and 3 into single-tube form and heated them to 60°C before initiating PCR procedures. Subsequent results also showed that fluorescence values and ct values of amplification curves were basically the same as those of the non-paraffin group, indicating that separating agents did not affect the performance of PCR.

Obviously, the above examples of the present invention are only instances for clearly illustrating the present invention, rather than limiting the embodiments of the present invention. For those of ordinary skill in the field, changes or modifications in other different forms may also be conducted on the basis of the above illustration. There is no need and cannot be exhaustive of all embodiments here.

## Claims

1. A separating agent suitable for a nucleic acid amplification system, comprising polyethylene wax, solid paraffin and liquid paraffin, **characterized in that** the polyethylene wax is polyethylene wax with a molecular weight of 4,000 to 6,000, the solid paraffin is solid paraffin with the carbon atom number of 20 to 25 and the liquid paraffin is liquid paraffin with the carbon atom number of 9 to 11.

2. The separating agent according to claim 1, wherein a mass ratio of the polyethylene wax to the solid paraffin to the liquid paraffin is: (0.02 - 0.05) : (0.6 - 1.2) : (4 - 10).

3. The separating agent according to claim 2, wherein a mass ratio of the polyethylene wax to the solid paraffin to the liquid paraffin is: (0.03 - 0.05) : (0.6 - 0.8) : (4 - 10).

4. A method for separating a nucleic acid amplification system, **characterized in that**, the separating agent of any of claims 1 to 3 is utilized as a separating layer to separate the nucleic acid amplification system in a same container; and the separating layer is ruptured by applying an external force, so as to realize mixing of the nucleic acid amplification system.

5. The method according to claim 4, wherein the external force is a centrifugal force.

6. The method according to claim 4, wherein the applying the external force is to centrifuge for 5 to 60 seconds under a condition of a centrifugal force not less than 5,000 g.

7. The method according to claim 4, wherein the applying the external force is to centrifuge for 10 to 30 seconds at 5,000 g to 10,000 g, and preferably, the applying the external force is to centrifuge for 20 seconds at 5,000 g.

## Patentansprüche

1. Trennmittel, das für ein Nukleinsäureamplifikationssystem geeignet ist, umfassend Polyethylenwachs, festes Paraffin und flüssiges Paraffin, **dadurch gekennzeichnet, dass** das Polyethylenwachs Polyethylenwachs mit einem Molekulargewicht von 4,000 bis 6,000 ist, das feste Paraffin festes Paraffin mit der Kohlenstoffatom-Zahl von 20 bis 25 ist und das flüssige Paraffin flüssiges Paraffin mit der Kohlenstoffatom-Zahl von 9 bis 11 ist.

2. Trennmittel nach Anspruch 1, worin ein Massenverhältnis des Polyethylenwachses zum festen Paraffin zum flüssigen Paraffin (0.02 - 0.05) : (0.6 - 1.2) : (4 - 10) ist.

3. Trennmittel nach Anspruch 2, worin ein Massenverhältnis des Polyethylenwachses zum festen Paraffin zum flüssigen Paraffin (0.03 - 0.05) : (0.6 - 0.8) : (4 - 10) ist.

4. Verfahren zum Trennen eines Nukleinsäureamplifikationssystems, **dadurch gekennzeichnet, dass** das Trennmittel nach einem der Ansprüche 1 bis 3 als Trennschicht verwendet wird, um das Nukleinsäureamplifikationssystem in einem selben Behälter zu trennen; und die Trennschicht durch Aufbringung einer äußeren Kraft gebrochen wird, um das Mischen des Nukleinsäureamplifikationssystems zu verwirklichen.

5. Verfahren nach Anspruch 4, worin die äußere Kraft eine Zentrifugalkraft ist.

6. Verfahren nach Anspruch 4, worin das Aufbringen der äußeren Kraft darin besteht, für 5 bis 60 Sekunden unter einer Bedingung einer Zentrifugalkraft nicht kleiner als 5,000 g zu zentrifugieren.

7. Verfahren nach Anspruch 4, worin das Aufbringen der äußeren Kraft darin besteht, für 10 bis 30 Sekunden bei 5,000 g bis 10,000 g zu zentrifugieren, und vorzugsweise das Aufbringen der äußeren Kraft darin besteht, für 20 Sekunden bei 5,000 g zu zentrifugieren.

## Revendications

1. Agent de séparation adapté à un système d'amplification d'acide nucléique, comprenant de la cire de polyéthylène, de la paraffine solide et de la paraffine liquide, **caractérisé en ce que** la cire de polyéthylène est une cire de polyéthylène ayant un poids moléculaire de 4,000 à 6,000, la paraffine solide est une paraffine solide ayant un nombre d'atomes de carbone de 20 à 25 et la paraffine liquide est une paraffine liquide ayant un nombre d'atomes de carbone de 9 à 11.

2. Agent de séparation selon la revendication 1, dans lequel le rapport massique entre la cire de polyéthylène, la paraffine solide et la paraffine liquide est : (0.02 - 0.05) : (0.6 - 1.2) : (4 - 10).

3. Agent de séparation selon la revendication 2, dans lequel le rapport massique entre la cire de polyéthylène, la paraffine solide et la paraffine liquide est : (0.03 - 0.05) : (0.6 - 0.8) : (4 - 10).

4. Procédé de séparation d'un système d'amplification d'acide nucléique, **caractérisé en ce que** l'agent de séparation de l'une quelconque des revendications 1 à 3 est utilisé comme couche de séparation pour séparer le système d'amplification d'acide nucléique dans un même récipient ; et la couche de séparation est rompue par application d'une force externe, de manière à réaliser le mélange du système d'amplification d'acide nucléique.

5. Procédé selon la revendication 4, dans lequel la force externe est une force centrifuge.

6. Procédé selon la revendication 4, dans lequel l'application de la force externe consiste à centrifuger pendant 5 à 60 secondes dans des conditions de force centrifuge non inférieure à 5,000 g.

7. Procédé selon la revendication 4, dans lequel l'application de la force externe consiste à centrifuger pendant 10 à 30 secondes à 5,000 g à 10,000 g, et de préférence, l'application de la force externe consiste à centrifuger pendant 20 secondes à 5,000 g.
